# EUROPÄISCHE PATENTSCHRIFT

(11) **EP 1 725 167 B1**
(45) Veröffentlichungstag und Bekanntmachung des Hinweises auf die Patenterteilung: **27.07.2011**
(21) Anmeldenummer: 05714931.2
(22) Anmeldetag: 04.02.2005
(51) Int. Cl.: A61B 5/15

(54) **BLUTENTNAHMEVORRICHTUNG FÜR NEUGEBORENE UND KLEINKINDER**
BLOOD-COLLECTION DEVICE FOR NEWBORN BABIES AND INFANTS
DISPOSITIF DE PRELEVEMENT D'ECHANTILLONS DE SANG POUR NOUVEAUX NES ET JEUNES ENFANTS

(30) Priorität: 17.03.2004 DE 102004013379
(43) Veröffentlichungstag der Anmeldung: 29.11.2006
(73) Patentinhaber: Sarstedt AG & Co., 51582 Nümbrecht (DE)
(72) Erfinder: SARSTEDT, Walter, 51588 Nümbrecht (DE)
(74) Vertreter: Grosse, Wolf-Dietrich Rüdiger
(86) Internationale Anmeldenummer: PCT/DE2005/000180
(87) Internationale Veröffentlichungsnummer: WO 2005/089650

(56) Entgegenhaltungen:
- EP-A- 0 732 077
- WO-A-92/20281
- DE-A1- 3 802 353
- US-A- 4 844 089
- US-A- 5 086 782

## Beschreibung

Die Erfindung betrifft eine Blutentnahmevorrichtung für Neugeborene und Kleinkinder, umfassend eine mit einem Bluteinlaß und -auslaß versehene, in einem Halter mit Griffbereich angeordnete Kanüle.

Eine solche Vorrichtung zur Entnahme von venösem Blut bei Neu- bzw. Frühgeborenen, Säuglingen und Kleinkindern ist durch die DE 100 60 302 A1 bekanntgeworden. Die an ihrem distalen Ende mit einer angeschärften Spitze ausgebildete Hohlnadel bzw. Kanüle weist an ihrem hinteren, proximalen Ende einen seitlich zu ihrer Längsachse abgewinkelten Blutauslaß auf und ist in einem Halter angeordnet, dessen hinter dem Blutauslaß liegender Griffbereich eine Anfass- bzw. Führungsmöglichkeit bietet. Bei der Blutentnahme wird unter die Auslassöffnung der Kanüle ein das ausströmende Blut auffangendes Gefäß gehalten.

Anders als bei herkömmlich eingesetzten Injektionsnadeln braucht die Kanüle dieser Blutentnahmevorrichtung nicht mehr besonders präpariert zu werden, z. B. ist kein Abbrechen eines ansonsten üblichen Luer-Konus mehr erforderlich, wodurch das Infektionsrisiko beim Patienten und ein Verletzungs- und Infektionsrisiko bei der das Blut abnehmenden Person verringert werden. Allerdings ist eine visuelle Kontrolle der zu entnehmenden Blutmenge nur sehr unzureichend möglich, weil bei diesen Blutentnahmevorrichtungen von nur geringer Größe das Griffstück und/oder die Finger einer das Blut entnehmenden Person die Sicht auf die Auslassöffnung erheblich einschränken, möglicherweise sogar völlig verdecken. Die seitlich abgewinkelte Auslassöffnung führt außerdem dazu, dass die Kanüle nur in einer Gebrauchslage einen optimalen Blutauslaß ermöglicht. Abgesehen davon, dass die Herstellung einer gekrümmten, seitlich abgewinkelten Kanüle aufwendig ist, liegt zudem auch noch der Nachteil vor, dass der durch die Krümmung bedingte höhere Strömungswiderstand der Kanüle den Blutfluß des ohnehin nur in einer geringen Menge entnommenen Blutes behindert.

Der Erfindung liegt daher die Aufgabe zugrunde, eine gattungsgemäße Blutentnahmevorrichtung zu schaffen, mit der sich die genannten Nachteile vermeiden lassen und die insbesondere die Anwender- und Bedienungsfreundlichkeit verbessert.

Diese Aufgabe wird erfindungsgemäß durch ein den Griffbereich mit dem Kanülenhalter verbindendes, bügelartiges Überbrückungselement gelöst. Hiermit lassen sich mehrere Vorteile gleichzeitig erreichen. Das Auslassende der Kanüle lässt sich in den ungehindert einsehbaren, freien Bereich des Überbrückungs- bzw. Überspannungselementes vorsehen. Damit lässt sich ausschließen, dass durch Sichtbeeinträchtigung von der geringen entnommenen Blutmenge ein Teil verloren geht. Da Einlaß- und Auslassöffnung der Kanüle auf einer Achse liegen, Krümmungen bzw. Abzweigungen somit vermieden sind, kommt es nicht zu Reibungsverlusten, die die Blutentnahme zusätzlich zum niedrigen Blutdruck und der geringen Blutmenge bei Früh- bzw. Neugeborenen, Säuglingen oder Kleinkindern erschweren könnten.

Das Überbrückungselement schafft weiterhin einen solchen Freiraum, der es ermöglicht, einen relativ großen Auffangbehälter unter die Auslassöffnung zu halten. Dies stellt sicher, dass auch kleinste Mengen Blutes ohne Verlust aufgefangen werden können. Der freie Blick auf die Auslassöffnung macht es auch möglich, sofort zu erkennen, wenn der erste Tropfen Blut fließt. Die Handhabung der erfindungsgemäßen Blutentnahmevorrichtung wird wesentlich vereinfacht und sicherer, denn die freie Sicht auf die Kanüle gewährleistet deren gezielte Einführung in die Vene. Weiterhin insbesondere auch, dass sich die Blutentnahmevorrichtung in der Vene problemlos drehen lässt, um einen optimalen Bluteinlaß zu erreichen und zu verhindern, dass sich die Venenwand auf die schräge Öffnung bzw. Anspitzung der Kanüle legt, womit diese verschlossen wäre. Trotz der geschaffenen Drehmöglichkeit ändert sich gleichwohl die Position der Auslassöffnung nicht, so dass ein optimaler Blutausfluß bei jeder Drehposition gewährleistet ist. Im Gegensatz hierzu bietet eine Kanüle mit einer Krümmung bzw. einer Abzweigung nur eine optimale Gebrauchslage, in der das Blut ausfließen kann. Alle anderen Lagen erschweren den Blutausfluß bzw. machen ihn unter Umständen sogar unmöglich, z.B, wenn die Auslassöffnung nach oben zeigt.

Weitere Merkmale und Einzelheiten der Erfindung ergeben sich aus dem Patentanspruch und der nachfolgenden Beschreibung eines in den Zeichnungen dargestellten Ausführungsbeispiels der Erfindung. Es zeigen:

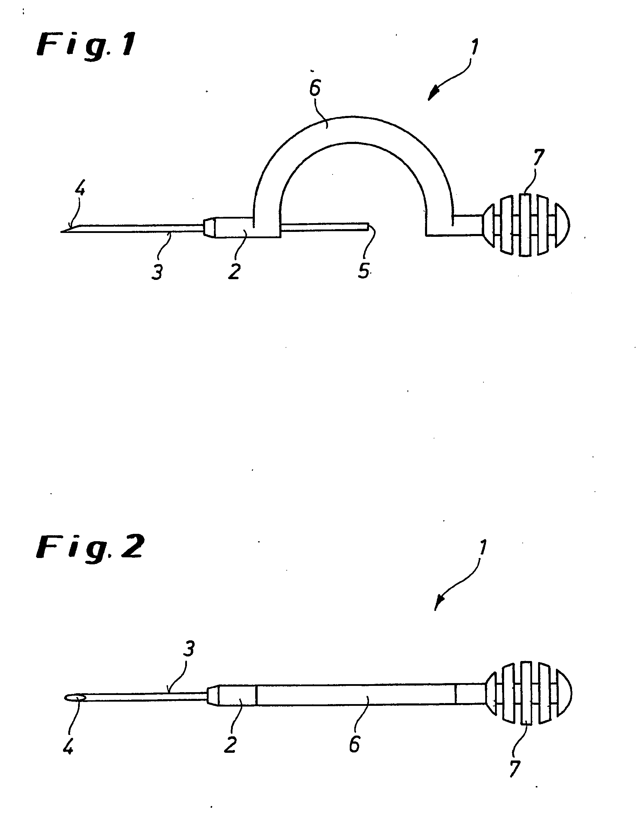

Fig. 1 eine Seitenansicht einer Blutentnahmevorrichtung; und
Fig. 2 die Blutentnahmevorrichtung nach Fig. 1 in der Draufsicht.

Eine in den Figuren dargestellte Blutentnahmevorrichtung 1 besitzt eine in einem Halter 2 angeordnete Hohlnadel bzw. Kanüle 3, die zum Einstechen in eine Vene an ihrem vorderen Ende mit einer angeschärften Spitze 4 als Bluteinlaß versehen ist und am entgegengesetzten, proximalen Ende einen Blutauslaß 5 bereitstellt. Der Blutauslaß 5 liegt in einem großen Freiraum, den ein bügelartiges Überbrückungselement 6 bietet, das den Kanülenhalter 2 mit einem Griffbereich 7 der Blutentnahmevorrichtung 1 verbindet.

Das überspannende Überbrückungselement 6 ermöglicht ein problemloses Drehen der Blutentnahmevorrichtung bei mit seiner Spitze 4 in eine Vene eingestochener Kanüle 3 und bietet einen großen, für eine Bedienungsperson frei einsehbaren Bereich mit der Möglichkeit, einen großen Auffangbehälter benutzen und schon gleich den ersten Tropfen fließenden Blutes erkennen zu können, so dass sich auch kleinste Mengen Blutes ohne Verlust auffangen lassen.

## Patentansprüche

1. Blutentnahmevorrichtung (1) für Neugeborene und Kleinkinder, umfassend eine mit einem offenen Bluteinlaß und -auslaß (4 bzw. 5) versehene, in einem Halter (2) mit Griffbereich (7) angeordnete Kanüle (3), **gekennzeichnet durch**
ein den Griffbereich (7) mit dem Kanülenhalter (2) verbindendes, bügelartiges, einen Freiraum für den Blutauslaß (5) zwischen dem Kanülenhalter (2) und dem Griffbereich (7) schaffendes Überbrückungselement (6).

## Claims

1. A blood collection device (1) for newborn babies and small children, comprising a cannula (3) which is provided with an open blood inlet and outlet (4 and 5) and is arranged in a holder (2) having a grip region (7), **characterised by** a bow-shaped bridging element (6) which connects the grip region (7) to the cannula holder (2) and creates a free space for the blood outlet (5) between the cannula holder (2) and the grip region (7).

## Revendications

1. Dispositif de prélèvement sanguin (1) pour nouveau-nés et enfants en bas âge comprenant une canule (3) munie d'une entrée et d'une sortie de sang ouvertes (4 respectivement 5), et disposée dans un support (2) avec une zone de préhension (7), **caractérisé par** un élément d'enjambement (6) reliant la zone de préhension (7) au support (2) de canule, en forme d'étrier, et créant un espace libre pour la sortie de sang (5) entre le support (2) de canule et la zone de préhension (7).
